# EUROPEAN PATENT SPECIFICATION

(11) **EP 2 719 405 B1**
(45) Date of publication and mention of the grant of the patent: **01.08.2018**
(21) Application number: 12797165.3
(22) Date of filing: 08.06.2012
(51) Int. Cl.: A61M 1/14

(54) **BLOOD PURIFICATION DEVICE**
BLUTREINIGUNGSVORRICHTUNG
DISPOSITIF DE PURIFICATION DU SANG

(30) Priority: 10.06.2011 JP 2011130278
(43) Date of publication of application: 16.04.2014
(73) Proprietor: Nikkiso Company Limited, Tokyo 150-6022 (JP)
(72) Inventor: FURUHASHI, Tomohiro, Makinohara-shi Shizuoka 421-0496 (JP); SUZUKI, Hiroaki, Makinohara-shi Shizuoka 421-0496 (JP); HIRANO, Takayuki, Makinohara-shi Shizuoka 421-0496 (JP); SUZUKI, Tomohiro, Makinohara-shi Shizuoka 421-0496 (JP)
(74) Representative: von Hirschhausen, Helge
(86) International application number: PCT/JP2012/064774
(87) International publication number: WO 2012/169609

(56) References cited:
- JP-A- 8 096 272
- JP-A- 2004 313 324
- JP-A- 2004 313 324
- JP-A- 2009 075 656
- US-B1- 6 971 072

## Description

### Technical Field

The present invention relates to a blood purification apparatus that performs blood purification treatment by purifying blood of a patient which is extracorporeally circulated, including a display device which performs a display relating to the blood purification treatment.

### Background Art.

A dialysis apparatus as a blood purification apparatus used in dialysis treatment is adapted such that a plurality of dialysis apparatuses are installed in a dialysis room disposed inside medical facilities such as hospitals and the dialysis treatment (blood purification treatment) is performed on a large number of patients inside the dialysis room. Such a dialysis apparatus generally has a display device arranged in order to perform a display relating to the dialysis treatment, and is configured to be capable of performing a predetermined operation relating to the dialysis treatment while viewing the display by using the display device. A plurality of screens displayed by using the display device are prepared in advance. Necessary information can be understood by switching the plurality of screens to be displayed.

Incidentally, since the dialysis treatment takes relatively long time be operated, it is difficult for a health care worker to always wait on the dialysis apparatus during an operation of the dialysis apparatus. In general, the health care worker performs the operation or work on the dialysis apparatus only when necessary such as when starting the operation of the dialysis apparatus, when an alarm is issued or when the operation is completed. During many other times, the dialysis apparatus is operated in such a manner that an operating status is confirmed by the health care worker who visits the dialysis room and visually checks the display on the display device from a relatively distant position.

In the related art, the dialysis apparatus has been proposed which includes the display device capable of switching a normal mode where an input operation relating to the dialysis treatment can be performed and the display relating to the dialysis treatment is performed and a display mode where the display relating to the dialysis treatment is exclusively performed. That is, if it is detected that the health care worker is close to the dialysis apparatus, the display device is placed in a state which enables the operation or the work as the normal mode. If it is determined that the health care worker is not close to the dialysis apparatus, the display device is automatically switched over to the display mode.

In JP 2004 313324 A a display system for a hemodialysis apparatus is described, wherein the display system is capable of providing on a screen different background colors for each process.

In US 6,971,072 B1 a method for controlling one user interface associated with a device is described. The presentation of the data varies as a function of changes in the sensed conditions such that the presentation mode corresponds to the sensed conditions in a manner which enhances the ability of a user to perceive the data.

The display device included in the dialysis apparatus in the related art is set so as to display a layout placing emphasis on operability in the normal mode, and so as to display with emphasis on visibility from a distant position in the display mode. Accordingly, the normal mode and the display mode are different from each other in a display form (not only display content but also a layout and a form of each display). Since the related art does not pertain to the invention described in a publication, there is no information on related art documents to be described.

### Summary of Invention

### Technical Problem

However, in the blood purification apparatus in the related art, the display form using the display device is significantly different between the display during the normal mode and the display during the display mode. Accordingly, it is necessary to confirm an operation state of the display after becoming familiar with the difference between the display during the normal mode and the display during the display mode. Thus, there has been a problem that an unfamiliar person may feel difficulties in confirmation work. In particular, when switching the normal mode and the display mode, the mode is switched over to a significantly different display form in a short time. Consequently, it has been difficult to carry out smooth and excellent confirmation work.

The present invention is made in view of such circumstances, and aims to provide a blood purification apparatus which can maintain operability during a normal mode and visibility during a display mode, and which can smoothly and excellently confirm an operation status in any case of the normal mode and the display mode.

### Solution to Problem

According to the invention described in Claim 1, there is provided a blood purification apparatus that performs blood purification treatment by purifying blood of a patient which is extracorporeally circulated, including a display device which performs a display relating to the blood purification treatment. The display device can switch a normal mode where an input operation relating to the blood purification treatment can be performed and a display relating to the blood purification treatment is performed and a display mode where the display relating to the blood purification treatment is exclusively performed. In the display during the display mode, a layout and a form of the display during the normal mode are maintained and enlargedly displayed. When it is detected that the health care worker is close to the dialysis apparatus by using a detection sensor, when there is the detection by the detection sensor, the mode is switched over to normal mode, and when there is no detection by the detection sensor for a certain period of time, the mode is switched over to the display mode.

According to the invention described in Claim 2, in the blood purification apparatus described in Claim 1, the display device is a touch panel which enables a predetermined input by touching a switch unit displayed in a desired position on a screen, and an input operation during the normal mode can be performed using the switch unit.

According to the invention described in Claim 3, in the blood purification apparatus described in Claim 2, the display device omits displaying the switch unit when the normal mode is switched over to the display mode.

According to the invention described in Claim 4, in the blood purification apparatus described in any one of Claims 1 to 3, the display device automatically switches the display mode over to the normal mode under a condition that an alarm is issued.

According to the invention described in Claim 5, in the blood purification apparatus described in any one of Claims 1 to 4, the display device gradually changes a size of the display in a switching process between the normal mode and the display mode.

### Advantageous Effects of Invention

According to the invention described in Claim 1, in the display during the display mode, the layout and the form of the display during the normal mode are maintained and enlargedly displayed. Therefore, the operability during the normal mode and the visibility during the display mode can be maintained, and it is possible to smoothly and excellently confirm an operation status in any case of the normal mode and the display mode.

According to the invention described in Claim 2, the display device is a touch panel which enables the predetermined input by touching the switch unit displayed in the desired position on the screen, and the input operation during the normal mode can be performed using the switch unit. Therefore, it is possible to eliminate a need for an operating device separated from the display device, thereby enabling a configuration of the blood purification apparatus to be simplified.

According to the invention described in Claim 3, the display device omits to display the switch unit when the normal mode is switched over to the display mode. Therefore, it is possible to enlarge the display during the display mode by using the omitted space, thereby enabling the visibility to be further improved.

According to the invention described in Claim 4, the display device automatically switches the display mode over to the normal mode under the condition that the alarm is issued. Therefore, it is possible to quickly and smoothly find and cope with a cause for the alarm issuing.

According to the invention described in Claim 5, the display device gradually changes a size of the display in a switching process between the normal mode and the display mode. Therefore, it is possible to reliably understand a corresponding relationship of the display in switching between the normal mode and the display mode.

### Brief Description of Drawings

[Fig. 1] Fig. 1 is a front view illustrating a blood purification apparatus according to an embodiment of the present invention.
[Fig. 2] Fig. 2 is a schematic diagram illustrating a screen during a normal mode which is displayed by a display device in the blood purification apparatus.
[Fig. 3] Fig. 3 is a schematic diagram illustrating a screen during a display mode which is displayed by the display device in the blood purification apparatus.

### Description of Embodiments

Hereinafter, an embodiment of the present invention will be described in detail with reference to the drawings.

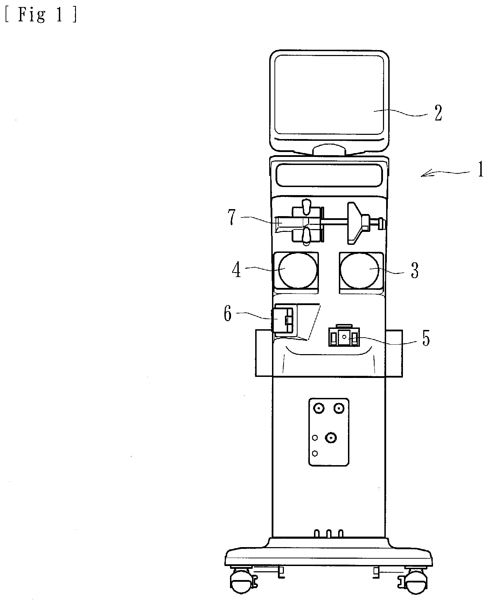

A blood purification apparatus according to the present embodiment is applied to a hemodialysis apparatus that performs dialysis treatment by purifying blood of a patient which is extracorporeally circulated. As illustrated in Fig. 1, the blood purification apparatus is mainly configured to include a dialysis device 1 (dialysis monitoring device) having a blood pump 3, a substitution pump 4, a bypass connector receiver 5, a bubble detector 6 and an infusion pump 7; and a display device 2 which is arranged above the dialysis device 1 and performs a display relating to blood purification treatment.

In the dialysis device 1, pipes such as a dialysate introduction line and a dialysate discharge line (not illustrated) are formed. A dialyzer (blood purifier) is connected to the dialysate introduction line and the dialysate discharge line, and a blood circuit is connected to the dialyzer. The blood circuit can extracorporeally circulate the blood of the patient and the dialyzer can purify the blood which is extracorporeally circulated.

The blood pump 3 is attached to the blood circuit (artery side blood circuit) and causes the blood of the patient to flow through a flexible tube configuring the blood circuit. The substitution pump 4 causes a substitution to flow inside the blood circuit through a tube for the substitution. In addition, the bubble detector 6 detects a bubble inside the blood circuit. The infusion pump 7 drives a syringe containing an anticoagulant (heparin) for example and continuously infuses the anticoagulant into the blood circuit.

In addition, the bypass connector receiver 5 receives and holds a bypass connector. When the blood purification treatment is not performed, the bypass connector is connected to a tip of the dialysate introduction line (dialysate exit port) and a tip of the dialysate discharge line (dialysate returning port), and can short-circuit (connects the tips to each other) the dialysate introduction line and the dialysate discharge line. When short-circuiting the dialysate introduction line and the dialysate discharge line, the bypass connector is held by the bypass connector receiver 5. Therefore, if the bypass connector is held by the bypass connector receiver 5, a switch reacts, thereby enabling detection that the dialysate introduction line and the dialysate discharge line are short-circuited.

The display device 2 is a touch panel (for example, touch panel such as a color LCD) which enables a predetermined input by touching a switch unit displayed in a desired position on a screen. The display device 2 can display various information items relating to the dialysis treatment (blood purification treatment) by switching a plurality of screens, and can perform a predetermined operation through an input operation by touching a predetermined switch unit displayed on a predetermined screen (in the present embodiment, screen during a normal mode).

In addition, the display device 2 can switch the normal mode which enables the input operation relating to the dialysis treatment and performs the display relating to the dialysis treatment and a display mode where the display relating to the dialysis treatment is exclusively performed. That is, when the dialysis apparatus is operated, if it is detected that a health care worker is close to the dialysis apparatus, the display device 2 is automatically switched over to the normal mode so as to perform the display placing an emphasis on operability, and if it is determined that the health care worker is not close to the dialysis apparatus, the display device 2 is automatically switched over to the display mode so as to perform the display placing an emphasis on visibility from a distant position.

In order to understand whether or not the health care worker is close to the dialysis apparatus, the following method may be considered. If it is detected that the health care worker is close to the dialysis apparatus by using a detection sensor such as an optical sensor, when there is the detection by the detection sensor, it is possible to switch the mode over to normal mode, and when there is no detection by the detection sensor for a certain period of time, it is possible to switch the mode over to the display mode. In addition, when there is the input operation with respect to the dialysis apparatus (in the present embodiment, input operation with respect to the touch panel as the display device), the mode may be switched over to the normal mode, and when there is no input operation for a certain period of time, the mode may be switched over to the display mode.

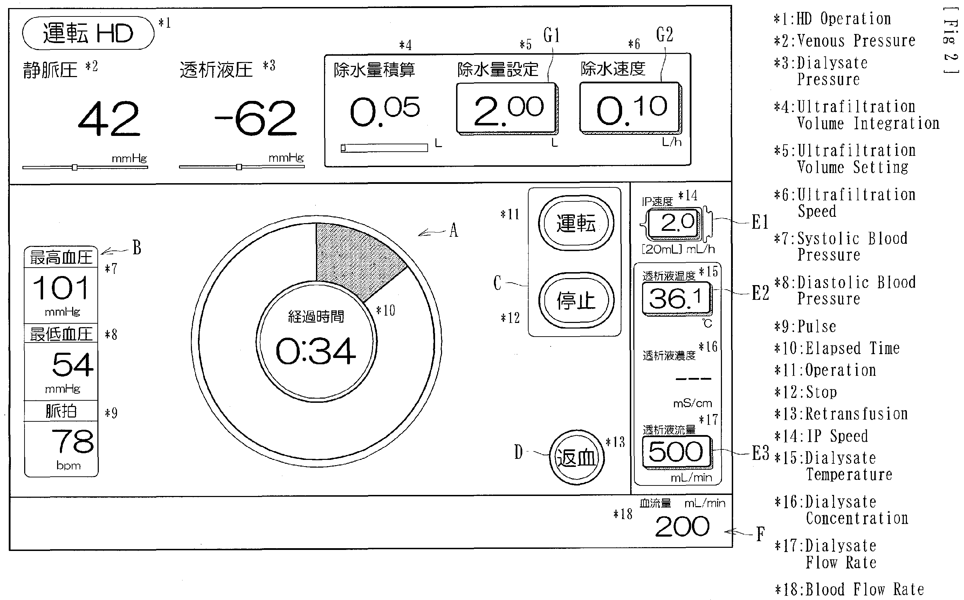

In the normal mode, as illustrated in Fig. 2, the following items can be displayed on a screen of the display device 2. That is, the display device 2 can display icons for various operations, such as an operation status (in a HD operation), various pressure values (venous pressure and dialysate pressure), ultrafiltration conditions (ultrafiltration volume integration, ultrafiltration volume setting and ultrafiltration speed), dialysate conditions (dialysate temperature, dialysate concentration and dialysate flow rate), an anticoagulant infusion speed (IP speed), a blood flow rate, elapsed time of dialysis treatment, and blood pressure / pulse values.

More specifically, the display device 2 during the normal mode displays, respectively on a predetermined portion on an identical screen, a graph display unit A displaying elapsed time of the dialysis treatment and the remaining time of the dialysis treatment; a display unit B displaying numerical values indicating a systolic blood pressure, a diastolic blood pressure and pulses; a switch unit C for operating or stopping the dialysis apparatus; a switch unit D for performing a retransfusion operation; a display unit E displaying a numerical value indicating an anticoagulant infusion speed (IP speed); a display unit E2 displaying a numerical value indicating a dialysate temperature; a display unit E3 displaying a numerical value indicating a dialysate flow rate; a display unit F displaying a numerical value of a blood flow rate; a display unit G1 displaying a setting value of an ultrafiltration volume; and a display unit G2 displaying a setting value of an ultrafiltration speed. In the present embodiment, in addition to the above-described display units, the display device 2 displays an operation status (in a HD operation), various pressure values (venous pressure and dialysate pressure), or a numerical value indicating ultrafiltration volume integration together.

Furthermore, in the present embodiment, the display units E1, E2, E3, G1 and G2 respectively display a predetermined numerical value, and enable a predetermined input operation by touching a portion of the units. Thus, the display units E1, E2, E3, G1 and G2 enable information to be understood by using the displayed numerical values, and in order to enable a visual check whether or not the input operation can be performed, a stereoscopic display (display where frames surrounding the numerical values are shaded) is performed. That is, the display units E1, E2, E3, G1 and G2 which are displayed during the normal mode also serve as a switch unit which enables a predetermined input operation.

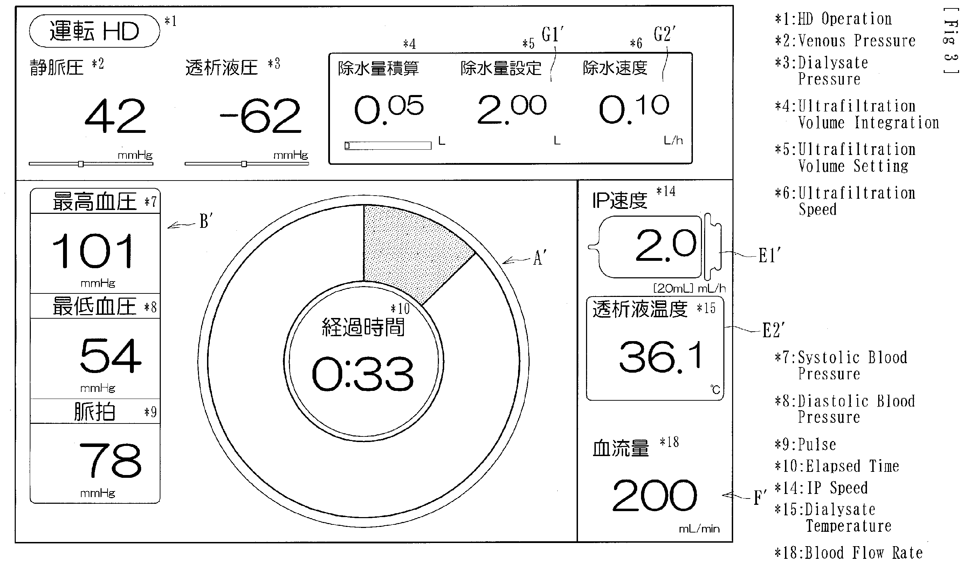

Here, as illustrated in Figs. 2 and 3, the display device 2 according to the present embodiment maintains and enlargedly displays a layout and a form of the display during the normal mode (refer to Fig. 2) for the display during the display mode (refer to Fig. 3). That is, during the display mode, the display device 2 displays a graph display unit A' formed from a display larger than that of the graph display unit A; a display unit B' formed from a display larger than that of the display unit B; display units E1' and E2' formed from a display larger than that of the display units E1 and E2; and a display unit F' formed from a display larger than that of the display unit F together on the identical screen while respectively maintaining the layout and the form.

In addition, during the display mode, the display device 2 performs the display relating to the hemodialysis treatment exclusively. For example, when the mode is switched over to the display mode, the display units E1, E2, E3, G1 and G2 during the normal mode have the stereoscopic displays disappeared, thereby displaying the numerical values only. The display units E1', E2', E3', G1' and G2' after the mode is switched over to the display mode are configured such that the input operation cannot be performed even by touching the numerical values.

In this manner, in the present embodiment, if the normal mode is switched over to the display mode, the layout (relative position with respect to the screen of each display) and the form of the display during the normal mode are maintained and enlargedly displayed (similarly enlarged display). On the other hand, if the display mode is switched over to the normal mode, the layout (relative position with respect to the screen of each display) and the form of the display during the display mode are maintained and contractedly displayed (similarly contracted display).

In particular, in the present embodiment, in a switching process between the normal mode and the display mode, a size of the display using the display device 2 can be set so as to be gradually changed. This enables reliable understanding of a corresponding relationship during the switching between the normal mode and the display mode. That is, if the size of the display is set so as to be gradually changed, compared to a case of being instantaneously changed, it is possible to follow a line of sight with respect to the display unit, thereby enabling the corresponding relationship to be clearly understood.

Furthermore, in the present embodiment, when the normal mode is switched over to the display mode, the display device 2 is configured to omit to display the switch unit. Specifically, after the mode is switched over to the display mode, the switch units C and D during the normal mode are set so as to be omitted without being displayed on the screen of the display device 2. As described above, if the mode is switched over to the display mode, the display units E1, E2, E3, G1 and G2 during the normal mode have the stereoscopic displays disappeared, and do not have a function as a "switch unit" which enables the input operation.

In the normal mode, a display which improves operability relating to the dialysis treatment is performed by displaying the switch unit to enable the input operation and by displaying much more information relating to the input operation. In the display mode, a display which improves visibility during rounds for example is performed by omitting the switch unit or the information relating to the input operation and by enlarging the display of the display unit (particularly the numerical value).

Furthermore, the display device 2 according to the present embodiment is configured such that the display mode is automatically switched over to the normal mode under a condition that an alarm is issued. The alarm is issued to call attention if the blood purification apparatus detects an abnormality, such as when work relating to the dialysis treatment (operation for the dialysis treatment and operation for preparing the treatment) is carried out, when there is an operation mistake or erroneous setting, or for example, when parameters of the venous pressure and ΔBV which are monitored (monitored values) are beyond a preset range. A device for issuing the alarm may have any form, either those which output an alarm sound using a speaker or those which turn on a warning lamp.

According to the above-described embodiment, the display during the display mode is performed by maintaining and enlargedly displaying the layout and the form of the display during the normal mode. Therefore, it is possible to maintain the operability during the normal mode and the visibility during the display mode, and it is possible to smoothly and excellently confirm the operation status in any case of the normal mode and the display mode. In the display mode, it is possible to more easily and visually check the numerical values and characters from a distant place by causing an inner portion of a display frame and a background in the display unit to have the same color as each other and by causing the numerical values or the characters and the background in the display unit to have a color different from each other.

In addition, when the normal mode is switched over to the display mode, the display device 2 omits to display the switch unit. Therefore, it is possible to enlarge the display during the display mode by using the omitted space, thereby enabling the visibility to be further improved. Further, the display device 2 automatically switches the display mode over to the normal mode under the condition that the alarm is issued. Therefore, it is possible to quickly and smoothly find and cope with the cause for the alarm issuing.

In addition, in the normal mode and the display mode, display content using the display device 2 is not limited to the present embodiment, but the display device 2 may perform various displays relating to the blood purification treatment. In the present embodiment, the display device 2 is applied to the hemodialysis apparatus in a hemodialysis (HD). However, instead of this, the display device 2 may be applied to a blood purification apparatus which can perform the other blood purification treatment (for example, treatment using hemodiafiltration (HDF), hemofiltration (HF) or slow continuous hemodiafiltration (CHF)).

### Industrial Applicability

If a display device can switch a normal mode which enables an input operation relating to blood purification treatment and performs a display relating to the blood purification treatment and a display mode which performs a display relating to the blood purification treatment exclusively, and if in the display during the display mode, a blood purification apparatus maintains and enlargedly display a layout and a form of the display during the normal mode, the display device can be applied to devices of various shapes and sizes, devices with other additional functions, and the like.

### Reference Sign List

1 dialysis device
2 display device (touch panel)
3 blood pump
4 substitution pump
5 bypass connector receiver
6 bubble detector
7 infusion pump

## Claims

1. A blood purification apparatus (1) that performs blood purification treatment by purifying blood of a patient which is extracorporeally circulated, comprising:
a display device (2) which performs a display relating to the blood purification treatment,
**characterized in that**
the display device (2) can switch a normal mode where an input operation relating to the blood purification treatment can be performed and a display relating to the blood purification treatment is performed and a display mode where the display relating to the blood purification treatment is exclusively performed, and
in the display during the display mode, a layout and a form of the display during the normal mode are maintained and enlargedly displayed, and
when it is detected that the health care worker is close to the dialysis apparatus (1) by using a detection sensor, when there is the detection by the detection sensor, the mode is switched over to normal mode, and when there is no detection by the detection sensor for a certain period of time, the mode is switched over to display mode.

2. The blood purification apparatus (1) according to claim 1,
wherein the display device (2) is a touch panel which enables a predetermined input by touching a switch unit displayed in a desired position on a screen, and
wherein an input operation during the normal mode can be performed using the switch unit.

3. The blood purification apparatus (1) according to claim 2,
wherein the display device (2) omits to display the switch unit when the normal mode is switched over to the display mode.

4. The blood purification apparatus (1) according to any one of claims 1 to 3,
wherein the display device (2) automatically switches the display mode over to the normal mode under a condition that an alarm is issued.

5. The blood purification apparatus (1) according to any one of claims 1 to 4,
wherein the display device (2) gradually changes a size of the display in a switching process between the normal mode and the display mode.

## Patentansprüche

1. Blutreinigungsvorrichtung (1), die eine Blutreinigungsbehandlung durchführt, indem das Blut eines Patienten, das extrakorporal im Kreislauf geführt wird, gereinigt wird, aufweisend:
eine Anzeigevorrichtung (2), die eine Anzeige bezüglich der Blutreinigungsbehandlung bewerkstelligt,
**dadurch gekennzeichnet, dass**
die Anzeigevorrichtung (2) zwischen einem normalen Modus, in dem ein Eingabevorgang bezüglich der Blutreinigungsbehandlung durchgeführt werden kann und eine Anzeige bezüglich der Blutreinigungsbehandlung bewerkstelligt wird, und einem Anzeigemodus, in dem ausschließlich die Anzeige bezüglich der Blutreinigungsbehandlung bewerkstelligt wird, umschalten kann und
in der Anzeige während des Anzeigemodus die Gestaltung und Form der Anzeige während des normalen Modus aufrechterhalten und vergrößert angezeigt werden, und
wenn unter Verwendung eines Detektionssensors detektiert wird, dass sich medizinisches Personal in der Nähe der Dialysevorrichtung (1) befindet, wenn die Detektion durch den Detektionssensor erfolgt, der Modus in den normalen Modus umgeschaltet wird, und, wenn für einen gewissen Zeitraum keine Detektion durch den Detektionssensor erfolgt, der Modus in den Anzeigemodus umgeschaltet wird.

2. Blutreinigungsvorrichtung (1) gemäß Anspruch 1,
wobei die Anzeigevorrichtung (2) ein Touchscreen ist, welcher eine vorbestimmte Eingabe durch Berühren einer Schaltfeldeinheit ermöglicht, die in einer gewünschten Position auf einem Schirm angezeigt wird, und
wobei ein Eingabevorgang während des normalen Modus unter Verwendung der Schaltfeldeinheit durchgeführt werden kann.

3. Blutreinigungsvorrichtung (1) gemäß Anspruch 2,
wobei die Anzeigevorrichtung (2) die Schaltfeldeinheit nicht anzeigt, wenn aus dem normalen Modus in den Anzeigemodus geschaltet wird.

4. Blutreinigungsvorrichtung (1) gemäß einem der Ansprüche 1 bis 3,
wobei die Anzeigevorrichtung (2) automatisch aus dem Anzeigemodus in den normalen Modus schaltet, wenn ein Alarm ausgegeben wird.

5. Blutreinigungsvorrichtung (1) gemäß einem der Ansprüche 1 bis 4,
wobei die Anzeigevorrichtung (2) allmählich die Größe der Anzeige bei einem Umschaltprozess zwischen dem normalen Modus und dem Anzeigemodus verändert.

## Revendications

1. Appareil de purification de sang (1) effectuant un traitement de purification de sang en purifiant le sang d'un patient, le sang étant circulé de façon extracorporelle, comprenant :
un dispositif d'affichage (2) qui est destiné à effectuer un affichage concernant le traitement de purification de sang,
**caractérisé en ce que**
le dispositif d'affichage (2) peut changer entre un mode normal, dans lequel une opération d'entrée concernant le traitement de purification de sang peut être effectué et un affichage concernant le traitement de purification de sang est effectué, et un mode d'affichage, dans lequel l'affichage concernant le traitement de purification de sang est effectué de façon exclusive, et
dans l'affichage pendant le mode d'affichage, la présentation graphique et la forme de l'affichage utilisées pendant le mode normal sont maintenues et affichées de façon agrandie, et
lorsque la détection que le travailleur de soins de santé est à proximité de l'appareil de dialyse (1) se fait en utilisant un capteur de détection, quand il y a une détection par le capteur de détection, le mode est changé au mode normal, et quand il n'y a pas de détection par le capteur de détection pendant un certain temps, le mode est changé au mode d'affichage.

2. Appareil de purification de sang (1) selon la revendication 1, dans lequel
le dispositif d'affichage (2) est un écran tactile qui permet une entrée prédéterminée en touchant une unité de commutation qui est affichée à une position souhaitée sur un écran, et une opération d'entrée pendant le mode normal peut être effectuée en utilisant ledit unité de commutation.

3. Appareil de purification de sang (1) selon la revendication 2, dans lequel
le dispositif d'affichage (2) omet d'afficher l'unité de commutation si le mode normal est changé au mode d'affichage.

4. Appareil de purification de sang (1) selon l'une quelconque des revendications 1 à 3, dans lequel
le dispositif d'affichage (2) est destiné à changer automatiquement du mode d'affichage au mode normal à condition qu'une alarme est émise.

5. Appareil de purification de sang (1) selon l'une quelconque des revendications 1 à 4, dans lequel
le dispositif d'affichage (2) est destiné à changer graduellement la grandeur d'affichage dans un processus de changement entre le mode normal et le mode d'affichage.
